# EUROPEAN PATENT APPLICATION

(11) **EP 1 752 075 A1**
(43) Date of publication of application: **14.02.2007**
(21) Application number: 05745809.3
(22) Date of filing: 02.06.2005
(51) Int. Cl.: A47K 7/00

(54) **SANITARY THIN PAPER FOR DOMESTIC USE**

(30) Priority: 04.06.2004 JP 2004166516
(71) Applicant: DAIO PAPER CORPORATION, Shikokuchuo-shi, Ehime 799-0492 (JP)
(72) Inventor: ITOH, Saeko, Fujinomiya-shi, Shizuoka 4180038 (JP); HIRASAWA, Akira, Fujinomiya-shi, Shizuoka 4180038 (JP)
(74) Representative: Henkel, Feiler & Hänzel
(86) International application number: PCT/JP2005/010113
(87) International publication number: WO 2005/117673

(57) **Abstract**

[Problem] Lotion tissue paper made of thin paper impregnated with a moisturizing component, which is able to leave a skin moist to keep a moisturizing feeling in the skin and in which the moisture hardly transpires even under a dry condition. [Means for Resolution] Hygienic thin paper for household use made of thin paper impregnated with a moisturizing component, wherein at least one member selected from the group consisting of a natural moisturizing factor and an intercellular lipid of a stratum corneum is contained as the foregoing moisturizing component, and a difference in the moisture content between a lapse of 4 hours and a lapse of 8 hours when allowing to stand within a dryer set up at 105°C is not more than 5%. The foregoing component selected from the group consisting of a natural moisturizing factor and an intercellular lipid of a stratum corneum can be combined with, for example, at least one member of an amino acid, an amino acid salt, an amino acid derivative, and ceramide.

## Description

### Technical Field

The present invention relates to hygienic thin paper for household use made of thin paper impregnated with a moisturizing component.

### Background Art

In the case where running of a nose is caused due to a cold or hay fever, etc. or rhinitis or nasal congestion is generated, tissue paper is frequently used. On this occasion, a mucous membrane of the nose is inflamed to cause an unpleasant feeling such as a pain. Thus, in order to relieve this, there have hitherto been known hygienic thin papers for household use inclusive of lotion tissue papers made of thin paper blended with a moisturizing component such as polyhydric alcohols, for example, glycerin and propylene glycol, sorbitol, and glucose.

A variety of improvements have been applied to such lotion tissue paper. For example, in order that a sticky feeling may not remain on a product, the following Patent Document 1 discloses lotion tissue papers in which an emulsified solution containing an emollient in components thereof is uniformly coated in a prescribed area ratio on thin paper.

Furthermore, for such a purpose that blending components neither reduce a tensile strength of a product nor penetrate into a wrapping material, the following Patent Document 2 discloses lotion tissue papers treated with a plastic material capable of giving a smooth touch such as petrolactam.

Moreover, for the purpose of obtaining a refreshing feeling even by long-term use, etc., the following Patent Document 3 discloses lotion tissue papers in which a perfume component such as menthol is encapsulated in a microcapsule and the foregoing microcapsule is dispersed in a lotion and coated on thin paper.
Patent Document 1: JP-A-4-9121
Patent Document 2: JP-T-10-501854
Patent Document 3: JP-T-10-510839

### Disclosure of the Invention

### Problems that the Invention is to Solve

However, in conventional lotion tissue papers, though a product per se is certainly soft so that an unpleasant feeling such as a pain is not generated at the time of use, there was involved a problem that the moisture of a skin is absorbed during a long-term use so that the skin is rubbed down or inflamed or becomes rough, whereby amoisturizing feeling is not obtained. With respect to this problem, any countermeasure has not been taken in conventional products including the inventions of the foregoing Patent Documents 1 to 3.

Furthermore, in conventional products, there was involved a problem that under a readily dried condition such as the winter season, the moisture transpires so that the texture of tissue paper is deteriorated.

Then, a principal object of the invention is to provide hygienic thin paper for household use made of thin paper impregnated with a moisturizing component, which is able to leave a skin moist to keep a moisturizing feeling in the skin and in which the moisture hardly transpires even under a dry condition.

### Means for Solving the Problems

In order to solve the foregoing problems, the invention according to claim 1 is to provide hygienic thin paper for household use made of thin paper impregnated with a moisturizing component, characterized in that:
at least one member selected from the group consisting of a natural moisturizing factor and an intercellular lipid of a stratum corneum is contained as the foregoing moisturizing component.

In the invention as set forth in the foregoing claim 1, at least one member selected from the moisturizing component group consisting of a natural moisturizing factor and an intercellular lipid of a stratum corneum was contained as the foregoing moisturizing component alone or together with a moisturizing component such as polyhydric alcohols, for example, glycerin and propylene glycol, sorbitol, and glucose. The foregoing natural moisturizing factor, commonly known by NMF is a substance which is present in a stratum corneum positioned in the outermost side of a skin, is well compatible with water and keeps the moisture; the foregoing intercellular lipid of a stratum corneum is a lipid present between cells of a stratum corneum and is a substance having a nature of keeping the moisture, thereby not emitting it; and by impregnating hygienic thin paper with the moisturizing component consisting of these natural moisturizing factor and intercellular lipid of a stratum corneum and continuously using the hygienic thin paper, the moisture is gradually given to the skin, thereby leaving it moist. Furthermore, since the foregoing natural moisturizing factor and intercellular lipid of a stratum corneum have such a nature of not emitting the absorbed moisture, the amount of transpiration of the moisture from the hygienic thin paper for household use can be reduced even under a dry condition, and the moisture can be kept in the thin paper itself over a long period of time.

The invention according to claim 2 is to provide the hygienic thin paper for household use as set forth in claim 1, wherein a difference in the moisture content between a lapse of 4 hours and a lapse of 8 hours when allowing to stand within a dryer set up at 105°C is not more than 5%.

In the invention as set forth in the foregoing claim 2, the foregoing moisturizing component is contained such that a difference in the moisture content between a lapse of 4 hours and a lapse of 8 hours when allowing to stand within a dryer set up at 105°C is not more than 5%. That is, though an increase of the moisture transpiration rate is seen until the time has elapsed to some extent, by making the moisture transpiration rate not increase so much thereafter, the texture of the hygienic thin paper can be kept over a long period of time.

The invention according to claim 3 is to provide the hygienic thin paper for household use as set forth in any one of claims 1 to 2, wherein the foregoing component selected from the group consisting of a natural moisturizing factor and an intercellular lipid of a stratum corneum is a combination with at least one member of an amino acid, an amino acid salt, an amino acid derivative, and ceramide.

The invention according to claim 4 is to provide the hygienic thin paper for household use as set forth in any one of claims 1 to 2, wherein the foregoing component selected from the group consisting of a natural moisturizing factor and an intercellular lipid of a stratum corneum is a combination with at least one member of pyrrolidonecarboxylic acid, a sodium salt thereof, proline, and ceramide.

The invention according to claim 5 is to provide the hygienic thin paper for household use as set forth in any one of claims 1 to 4, wherein the moisture transpiration rate when allowing to stand within a dryer set up at 105°C for 8 hours or more is not more than 70% by weight of the weight of the whole of the moisturizing components.

As a standard of the moisture transpiration rate, it is desired that in the foregoingmoisturizing component consisting of a natural moisturizing factor and an intercellular lipid of a stratum corneum, the moisture transpiration rate when allowing to stand within a dryer set up at 105°C for 8 hours or more is not more than 70% by weight of the weight of the whole of the moisturizing components.

The invention according to claim 6 is to provide the hygienic thin paper for household use as set forth in any one of claims 1 to 5, wherein the moisturizing moisture is contained in an amount of 7.0% by weight or more based on the weight of the foregoing thin paper.

The invention according to claim 7 is to provide the hygienic thin paper for household use as set forth in any one of claims 1 to 6, wherein the moisturizing component of at least one of the foregoing group consisting of a natural moisturizing factor and an intercellular lipid of a stratum corneum is contained in a proportion of from 40 to 60% by weight based on the amount of the whole of the moisturizing components.

The invention according to claim 8 is to provide the hygienic thin paper for household use as set forth in any one of claims 1 to 7, wherein the foregoing thin paper has a basis weight of from 10 to 40 g/m².

### Advantages of the Invention

As described above in detail, according to the invention, hygienic thin paper for household use which is able to leave a skin moist to keep a moisturizing feeling in the skin and in which the moisture hardly transpires even under a dry condition can be obtained.

### Best Modes for Carrying Out the Invention

The invention will be hereunder specifically described in detail.

The invention is concerned with hygienic thin paper for household use impregnated with a moisturizing component, such as lotion tissue paper, in which at least one member selected from the group consisting of a natural moisturizing factor and an intercellular lipid of a stratum corneum is contained as the foregoing moisturizing component alone or in addition to a moisturizing component which has hitherto been used, such as polyhydric alcohols, for example, glycerin and propylene glycol, sorbitol, and glucose.

The foregoing natural moisturizing factor is commonly named as NMF (Natural Moisturizing Factor) and is a substance which is contained in a stratum corneum positioned in the outermost side of a skin. The natural moisturizing factor is well compatible with water, has a nature of keeping the moisture and takes on the responsibility of keeping the skin young and fresh. The foregoing NMF is made of an amino acid, a lactic acid, urea, a citric acid salt, and so on, and in the present tissue paper, of these components, an amino acid, an amino acid salt, and an amino acid derivative are suitably used. More specifically, proline, glycine, alanine, cerin, threonine, alginine, glutamic acid, leucine, isoleucine, valine, pyrrolidonecarboxylic acid (hereinafter referred to as PCA), a pyrrolidonecarboxylic acid salt, and so on are suitably used. In addition, in the present tissue paper, of these, ones having a cyclic structure, for example, PCA, a sodium salt thereof, and proline are preferably used. There has been obtained knowledge that when PCA or a salt thereof is used in combination with proline, not only moisturizing properties are improved, but an effect for preventing conduction is brought. Furthermore, since these materials have a cyclic structure, they are chemically structurally stable.

On the other hand, the foregoing intercellular lipid of a stratum corneum is a lipid present between cells of a stratum corneum of a skin and is a substance having a nature of keeping the moisture, thereby not emitting it likewise the foregoing NMF. Specific examples of this intercellular lipid of a stratum corneum include substances such as ceramide, cholesterol, and phospholipid. In the present tissue paper, of these components, ceramide is most suitably used because extraction, etc. is easy.

Since the foregoing natural moisturizing factor and intercellular lipid of a stratum corneum are excellent in moisturizing properties and are a component which a skin of a human being possesses, by continuously using the lotion tissue paper according to the invention, the moisture content of the skin is gradually improved, whereby it is able to leave the skin moist and to keep a moisturizing feeling. Furthermore, since the lotion tissue paper holds the moisture and does not emit it outside, the moisture hardly transpires even under a dry condition, and the tissue paper is free from deterioration of texture.

Incidentally, since the foregoing natural moisturizing factor and intercellular lipid of a stratum corneum are a substance capable of keeping moisturizing properties, they are used in other fields, for example, as a component of therapeutic drugs of atopic dermatitis, xeroderma of adult female, and senile xerosis in the medical field; a hair care component of shampoo in the cosmetic field; and so on.

On the other hand, as other moisturizing components than the foregoing natural moisturizing factor and intercellular lipid of a stratum corneum, for example, at least one member of polyhydric alcohols such as glycerin, diglycerin, propylene glycol, 1,3-butylene glycol, and polyethylene glycol; sugars such as sorbitol, glucose, xylitol, maltose, maltitol, mannitol, and trehalose; glucose based chemicals and derivatives thereof; higher alcohols such as cetanol, stearyl alcohol, and oleyl alcohol; and liquid paraffins can be arbitrarily combined and used. Furthermore, this can be blended with amino acids such as glycine, aspartic acid, alginine, alanine, and cysteine; vegetable extract oils such as aloe extract, sweet hydrangea extract, *Angelica keiskei* extract, *karin* extract, cucumber extract, field horsetail extract, tomato extract, wild rose extract, luffa extract, lily extract, and *Astragalus sinicus* extract; vegetable oils such as olive extract, jojoba oil, rosehip oil, almond oil, eucalyptus oil, avocado oil, camellia oil, soybeanoil, sunfloweroil, sesame oil, and evening primrose oil; vitamins; hydrolyzed collagen; hydrolyzed keratin; hydrolyzed silk; chitosan; urea; honey; royal jelly; sodium hyaluronate; ceramide; squalane; vaseline; and so on.

With respect to moisturizing performance of the foregoing lotion tissue paper impregnated with a moisturizing component, it is desired that a difference in the moisture content between a lapse of 4 hours and lapse of 8 hours when allowing to stand within a dryer set up at 105°C is not more than 5%, and preferably not more than 3%. That is, though the moisture transpiration rate increases until the time has elapsed to some extent, by making the moisture transpiration rate not increase so much thereafter, the texture of the hygienic thin paper can be kept over a long period of time. In the case where the foregoing difference in the moisture content exceeds 5%, the moisture transpiration gradually increases in a proportion to some extent or more, and therefore, such is not preferable. Incidentally, the foregoing moisture content (%) is determined from { [ (weight of thin paper when allowing to stand within a thermo-hygrostat chamber)-(weight of thin paper when allowing to stand within a dryer and elapsing for a prescribed time)] (amount of moisture transpiration)/(weight of thin paper when allowing to stand within a thermo-hygrostat chamber) × 100}.

Furthermore, with respect to the foregoing moisturizing performance of the lotion tissue paper, it is desired that the moisture transpiration rate when allowing to stand within a dryer set up at 105°C for 8 hours or more is not more than 70% by weight, and preferably not more than 50% by weight of the weight of the whole moisturizing components. When the foregoing moisture transpiration rate exceeds 70% by weight, the texture of the product is deteriorated so that an effect for moisturizing a skin is not obtained, too. In particular, in the case where the foregoing moisture transpiration rate is not more than 50% by weight, the texture can be remarkably continued as compared with conventional products.

Furthermore, in order to meet the foregoing difference in moisture content and moisture transpiration rate, it is desired that the moisturizing component of at least one of the foregoing group consisting of a natural moisturizing factor and an intercellular lipid of a stratum corneum is contained in a proportion of from 40 to 60% by weight, and preferably from 45 to 55% by weight based on the amount of the whole of the moisturizing components. In the case where the blending rate of the foregoing natural moisturizing factor and intercellular lipid of a stratum corneum is less than 40% by weight, the moisture transpiration rate becomes large so that not only sufficient moisturizing properties are not obtained, but the texture under a dry condition cannot be kept. On the other hand, in the case where the foregoing blending rate exceeds 60% by weight, though it is possible to design to sufficiently keep the moisturizing properties, since the product becomes excessively moist, there are caused various problems that not only a sticky feeling is generated rather than a moist feeling, but the manufacturing costs increase, and therefore, such is not preferable. In particular, when the foregoing blending rate falls within the range of from 45 to 55% by weight, not only moisture is properly given, but there are free from the foregoing deterioration in quality and increase of the costs, whereby the product is balanced among all of a use feeling, material quality and cost performance.

Now, though the foregoing thin paper is basically used as usual tissue paper and besides, includes toilet papers and so on, it is desired to use one having a basis weight falling within the range of from 10 to 40 g/m², and more desirably from 10 to 20 g/m². When the basis weight is less than 10 g/m², a number of pores are generated on the thin paper, leading to a large cause of surface deterioration. Conversely, when the basis weight exceeds 40 g/m², not only the whole of the paper becomes hard, but a stiff feeling is generated so that a touch becomes worse.

Furthermore, it is desired that the foregoing lotion tissue paper contains the moisturizing component in an amount of 7.0% by weight or more, preferably 9.0% by weight or more, and more preferably 1.0% by weight or more based on the weight of the thin paper. When the amount of the moisturizing component is less than 7.0% by weight based on the weight of the thin paper, the moisture transpires in an early stage so that it is difficult to obtain the desired effects of the invention, for example, an moisturizing effect and an effect for preventing the deterioration of texture. An upper limit of the foregoing content of the moisturizing component is not more than 20% by weight, and preferably not more than 15% by weight.

### Examples

### (1) Measurement of moisture content in skin:

Monitors whose skins around a nose were not extremely dry (from 33 to 37% in terms of skin moisture content) were extracted among female monitors who became habitual to frequently blow one's nose due to hay fever (30 times or more a day) and requested to use a product according to the invention having an NMF component (sodium PCA) blended in a proportion of 50% by weight of the whole of the moisturizing components (Example), a conventional product containing only a conventionally used glycerin based moisturizing component (Comparative Example 1) and a conventional product not containing a moisturizing component at all (Comparative Example 2); and a moisture content on the skin surface after a lapse of 2 weeks was measured, and its state was visually observed. The obtained results are shown in Table 1.

Incidentally, the measurement of the skin moisture content was carried out in such a manner that the monitors entered a thermo-hygrostat chamber (at 23°C and 50%) for 2 hours prior to the measurement, the skin state became in a stationary state, and thereafter, the moisture content in the side face of the nose was measured by a skin moisture checker (MY707S, manufactured by Scalar Corporation).

**[Table 1]**

| | Example | Comparative Example 1 | Comparative Example 2 |
|---|---|---|---|
| Prior to measurement | 32.20% | 33.90% | 34.10% |
| After two weeks | 36.50% | 31.10% | 25.60% |
| Visual observation of the state | The state that the skin surface is not inflamed and that the skin is not rubbed down and is glossy. | The state that though the skin surface is not inflamed, the skin is rubbed down and is dry. | The state that the skin surface is inflamed and that a rough feeling is generated. |

As is clear from the results of Table 1, in the conventional products, the skin moisture content is reduced two weeks after the use, whereas in the product according to the invention, the skin moisture content is inversely improved. Furthermore, in the observation of the state two weeks after the use, in the case of the conventional products, the skin on the skin surface causes rubbing down and inflammation, etc., whereas in the case of the product according to the invention, such problems are not caused, and by continuously continuing the use, the skin inversely becomes in a glossy state.

### (2) Measurement of moisture transpiration rate under a dry condition:

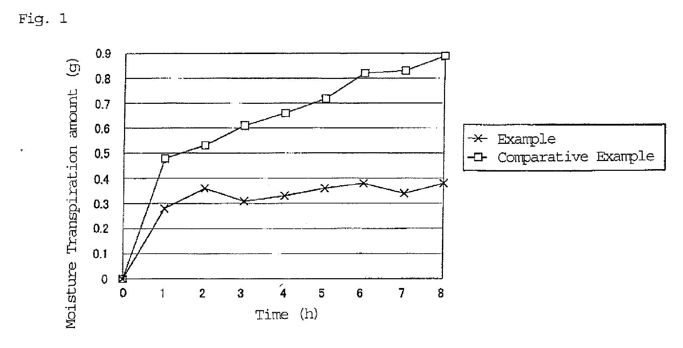

As shown in the following Table 2, a specimen containing each of a glycerin base chemical liquid and an NMF component (sodium PCA) in a ratio of 25% by weight based on the sheet weight (Example) and a specimen impregnated with a conventionally used glycerin base chemical liquid in a proportion of 50% by weight based on the sheet weight were prepared and allowed to stand in a thermo-hygrostat chamber for 18 hours, and then placed in a dryer set up at 105°C; and a weight reduction of a product weight of each sample from an initial product weight was measured every one hour, from which was then calculated a moisture transpiration amount. The measurement was carried out every one hour until a lapse of 8 hours. The obtained results are shown in Table 3 and a graph of Fig. 1.

**[Table 2]**

| | Unit | Example | Comparative Example |
|---|---|---|---|
| Sheet weight | g | 1.67 | 1.85 |
| Glycerin base chemical liquid | g | 0.41 | 0.825 |
| Sodium PCA | g | 0.42 | 0 |

**[Table 3]**

| | Example | | | Comparative Example | | |
|---|---|---|---|---|---|---|
| | Product weight (g) | Moisture transpiration amount (g) | Moisture transpiration rate (%) | Product weight (g) | Moisture transpiration amount (g) | Moisture transpiration rate (%) |
| At the time of start | 2.48 | 0.00 | 0.0 | 2.50 | 0.00 | 0.0 |
| After a lapse of 1 hour | 2.20 | 0.28 | 33.7 | 2.02 | 0.48 | 57.8 |
| After a lapse of 2 hours | 2.12 | 0.36 | 43.4 | 1.97 | 0.53 | 63.9 |
| After a lapse of 3 hours | 2.17 | 0.31 | 37.3 | 1.89 | 0.61 | 73.5 |
| After a lapse of 4 hours | 2.15 | 0.33 | 39.8 | 1.84 | 0.66 | 79.5 |
| After a lapse of 5 hours | 2.12 | 0.36 | 43.4 | 1.78 | 0.72 | 86.7 |
| After a lapse of 6 hours | 2.10 | 0.38 | 45.8 | 1.68 | 0.82 | 98.8 |
| After a lapse of 7 hours | 2.14 | 0.34 | 41.0 | 1.67 | 0.83 | 100.0 |
| After a lapse of 8 hours | 2.10 | 0.38 | 45.8 | 1.61 | 0.89 | 107.2 |

| | | | | | | |
|---|---|---|---|---|---|---|
| Note 1) Moisture transpiration rate (%) = [Moisture transpiration amount (g)] ÷ [Total addition amount of moisturizing component (g)] × 100 Note 2) A reason why in the Comparative Example, the moisture transpiration rate exceeds 100% after a lapse of 8 hours resides in the matter that the thin paper itself slightly contains moisture. | | | | | | |

As is clear from the results of Table 3 and Fig. 1, though in the case of the Comparative Example, the moisture entirely transpired, in the case of the product according to the invention, it was possible to suppress the moisture transpiration amount at not more than 50%.

Furthermore, in the foregoing Example and Comparative Example, the moisture content after a lapse of 4 hours and a lapse of 8 hours and a difference therebetween were as shown in the following Table 4.

**[Table 4]**

| | Moisture content after a lapse of 4 hours (%) | Moisture content after a lapse of 8 hours (%) | Difference in moisture content |
|---|---|---|---|
| Example | 13.3 | 15.3 | 2.0 |
| Comparative Example | 26.4 | 35.6 | 9.2 |

### Brief Description of the Drawing

Fig. 1 is a graph to show the relationship between a time for allowing to stand and a moisture transpiration amount in the Example and Comparative Example.

## Claims

1. Hygienic thin paper for household use made of thin paper impregnated with a moisturizing component, **characterized in that**:
at least one member selected from the group consisting of a natural moisturizing factor and an intercellular lipid of a stratum corneum is contained as said moisturizing component.

2. The hygienic thin paper for household use according to claim 1, wherein a difference in the moisture content between a lapse of 4 hours and a lapse of 8 hours when allowing to stand within a dryer set up at 105°C is not more than 5%.

3. The hygienic thin paper for household use according to any one of claims 1 to 2, wherein said component selected from the group consisting of a natural moisturizing factor and an intercellular lipid of a stratum corneum is a combination with at least one member of an amino acid, an amino acid salt, an amino acid derivative, and ceramide.

4. The hygienic thin paper for household use according to any one of claims 1 to 2, wherein said component selected from the group consisting of a natural moisturizing factor and an intercellular lipid of a stratum corneum is a combination with at least one member of pyrrolidonecarboxylic acid, a sodium salt thereof, proline, and ceramide.

5. The hygienic thin paper for household use according to any one of claims 1 to 4, wherein the moisture transpiration rate when allowing to stand within a dryer set up at 105°C for 8 hours or more is not more than 70% by weight of the weight of the whole of the moisturizing components.

6. The hygienic thin paper for household use according to any one of claims 1 to 5, wherein the moisturizing moisture is contained in an amount of 7.0% by weight or more based on the weight of the foregoing thin paper.

7. The hygienic thin paper for household use according to any one of claims 1 to 6, wherein the moisturizing component of at least one of said group consisting of a natural moisturizing factor and an intercellular lipid of a stratum corneum is contained in a proportion of from 40 to 60% by weight based on the amount of the whole of the moisturizing components.

8. The hygienic thin paper for household use according to any one of claims 1 to 7, wherein said thin paper has a basis weight of from 10 to 40 g/m².
